# FASCICULE DE BREVET EUROPEEN

(11) **EP 2 531 154 B1**
(45) Date de publication et mention de la délivrance du brevet: **22.06.2016**
(21) Numéro de dépôt: 11713035.1
(22) Date de dépôt: 03.02.2011
(51) Int. Cl.: A61F 9/00, B65D 47/18

(54) **FLACON DE CONDITIONNEMENT D'UN LIQUIDE À TÊTE DE DISTRIBUTION GOUTTE À GOUTTE**
FLÄSCHCHEN ZUR AUFBEWAHRUNG EINER FLÜSSIGKEIT MIT EINEM TROPFENAUSGABEKOPF
VIAL FOR PACKAGING A LIQUID HAVING A DRIP DISPENSING HEAD

(30) Priorité: 04.02.2010 FR 1000457
(43) Date de publication de la demande: 12.12.2012
(73) Titulaire: Laboratoires THEA, 63100 Clermont-Ferrand (FR)
(72) Inventeur: DEFEMME, Alain, F-63400 Chamalieres (FR); MERCIER, Fabrice, F-63000 Clermont-Ferrand (FR)
(74) Mandataire: Thibon, Norbert
(86) Numéro de dépôt international: PCT/IB2011/000182
(87) Numéro de publication internationale: WO 2011/095877

(56) Documents cités:
- WO-A1-95/28335
- DE-C- 904 267
- FR-A1- 2 770 495
- FR-A1- 2 872 137
- JP-A- 2006 213 332

## Description

La présente invention concerne la conception et la réalisation d'un récipient de conditionnement et de distribution contrôlée d'un liquide. Dans le cadre notamment d'une destination pour distribution en goutte à goutte, ses caractéristiques principales sont divulguées dans les revendications et concernent la tête de distribution du liquide à partir du réservoir d'un flacon à paroi à déformation élastiquement réversible revenant à sa conformation initiale par aspiration d'air vers l'intérieur du flacon après délivrance d'une dose de liquide.

L'invention s'applique de façon privilégiée, bien que non restrictive, au domaine des compositions liquides pharmaceutiques, notamment dans les applications ophtalmologiques. Il s'agit là d'un domaine où les questions de pureté du produit distribué et de précision de la distribution sont particulièrement importantes. Des besoins très proches peuvent se retrouver, par exemple, en cosmétologie ou dermatologie, notamment pour ce qui concerne la précision dans l'emplacement où amener la goutte expulsée, le cas échéant pour ce qui concerne le respect d'une dose d'administration prédéterminée.

L'invention a plus particulièrement pour objet comme indiqué dans les revendications, un flacon de conditionnement à réservoir à paroi à déformation élastiquement réversible et à tête de distribution de liquide par un embout compte-gouttes dépassant à l'extérieur du flacon qui comporte une membrane filtrante anti-bactérienne protégeant le liquide contenu dans le réservoir des agents polluants du milieu extérieur. Dans des flacons fabriqués industriellement par la Demanderesse, en conformité notamment avec la demande de brevet français publiée sous le numéro FR 2 872 137, qui divulgue les caractéristiques techniques placées dans le préambule de la revendication 1, une telle membrane est disposée entre le réservoir de liquide interne au flacon et l'embout de distribution, à la base du canal d'expulsion de gouttes, en travers du conduit de circulation de liquide, et c'est ce même conduit qui sert aussi à l'entrée de l'air extérieur venant regonfler le flacon en remplacement du liquide précédemment expulsé, après chaque opération de distribution.

Ce fonctionnement est rendu possible du fait que l'on utilise comme membrane filtrante antibactérienne une membrane bifonctionnelle, partiellement hydrophile et partiellement hydrophobe, du type de celles que l'on sait réaliser par exemple en polymère à base de polyéther sulfone. Le propre de ces membranes est de se laisser traverser en alternance tantôt préférentiellement par le liquide aqueux du flacon, tantôt préférentiellement par l'air en provenance de l'extérieur, en fonction d'un différentiel de pression entre la pression existante du côté de la membrane orienté vers le réservoir et la pression existante du côté opposé de la membrane orienté vers le canal de distribution.

La présence d'une telle membrane, autorisant d'une part le passage du liquide dans le sens de la distribution, sous l'effet d'une pression exercée par l'utilisateur sur la paroi du réservoir, et d'autre part le passage de l'air en sens inverse, de l'extérieur vers le réservoir, lorsque la pression sur le flacon est relâchée après la distribution d'une goutte de liquide, représente l'un des facteurs qui permettent de conditionner les liquides ophtalmiques dans des flacons multidoses sans qu'il soit besoin d'incorporer des agents conservateurs dans leur composition.

Mais le fonctionnement correct des flacons multidoses ainsi constitués demande un contrôle précis de la production des gouttes qui, dans les flacons réalisés par la Demanderesse et comme décrit dans le brevet déjà cité, passe par l'utilisation d'une tête de distribution présentant, en travers de la circulation du liquide entre le réservoir et la membrane, un insert à corps évidé contenant un tampon poreux (plus précisément microporeux) qui joue un rôle de régulateur du flux de liquide. Le tampon est à cet effet placé en aval du réservoir, dans le sens de la circulation du liquide depuis le réservoir vers l'embout de distribution, et en amont de la membrane bifonctionnelle. Il est avantageusement réalisé en un matériau à propriétés hydrophobes.

L'utilisation de tels flacons, si elle s'avère satisfaisante dans de nombreux cas, pose cependant des problèmes spécifiques de fonctionnement en goutte à goutte quand il s'agit de conditionner des solutions présentant des propriétés tensioactives. A tel point que dans ce cas, pour éviter le recours aux agents conservateurs on en vient à leur préférer les ampoules de conditionnement unidose, malgré leurs inconvénients en termes de consommation de produit (excédent de contenance restant perdu à chaque utilisation). Le problème de la mauvaise qualité de formation des gouttes se rencontre notamment pour des formulations ophtalmiques contenant soit des principes actifs qui sont par eux-mêmes à propriétés tensio-actives, soit des additifs tensio-actifs utilisés comme agents solubilisants (par exemple polysorbate, glycérol, polyéthylène-glycol ricinoléate), soit encore d'autres excipients, comme certains agents viscosants ou lubrifiants de la famille des dérivés polyvinyliques ou de celle des polyéthylène-glycols.

A l'origine de l'invention, il est apparu que la distribution en goutte-à-goutte dans de telles situations peut être gênée par le fait qu'il se forme de la mousse en sortie du tampon poreux et que le fonctionnement de la membrane bifonctionnelle hydrophile/hydrophobe s'en trouve perturbé, dès lors qu'un mélange biphasique liquide/gaz peut arriver à son contact.

Pour résoudre le problème et retrouver sensiblement la même efficacité en distribution goutte à goutte que celle que l'on apprécie dans le cas de liquides non moussants, on s'est alors attaché à travailler non plus sur les conditions de circulation du liquide lors de son expulsion du flacon, mais plutôt sur les conditions dans lesquelles s'effectue la rentrée d'air après chaque opération de distribution. C'est ainsi que l'on est parvenu à une forme de construction de la tête distribution qui tend à éviter la rétention d'air à l'intérieur du tampon poreux, l'air restant dans le tampon à chaque passage étant rendu responsable de la formation de mousse dans le liquide qui traverse ensuite le tampon en sens inverse.

Conformément à l'invention, il est proposé de conformer l'insert de la tête de distribution, qui par la périphérie de son corps principal se monte étanche dans le col du réservoir, avec une base prolongeant ledit corps au-delà du tampon poreux qui pénètre à l'intérieur du flacon en y ménageant des passages d'air répartis en étoile qui guident l'air radialement vers la périphérie du flacon. Tout en favorisant l'appel de l'air en extraction du tampon poreux, grâce à une section de passage largement ouverte ne provoquant pas de perte de charge en cet endroit, de tels passages ont pour effet de répartir l'air uniformément sur toute la section du flacon, au-dessus de la surface du liquide encore contenu dans le réservoir. La même base de l'insert forme au-delà de ces passages radiaux, une pastille centrale faisant obstacle à une projection d'air directement vers la surface du liquide dans l'axe de révolution général du flacon.

En complément de ce qui précède, l'invention prévoit avantageusement que dans le corps principal de l'insert, le tampon poreux soit distant de la membrane bifonctionnelle d'une hauteur axiale laissant à toute bulle d'air sortant du tampon toute latitude d'éclater avant de parvenir au niveau de la membrane, même dans le cas d'une formulation à propriétés tensio-actives. On évite ainsi tout risque de formation de mousse dans la goutte expulsée. On assuré qu'au moment de l'extraction d'une goutte, la membrane soit recouverte d'un film de liquide exempt d'air. On notera que la partie hydrophobe de la membrane représente alors le point critique, car à surfaces égales le débit d'air sur la partie hydrophobe est bien supérieur au débit de liquide de la partie hydrophile. Dans une formulation sans propriété tensioactive, les bulles d'air éclatent instantanément au moment de leur formation à la sortie du tampon poreux sous l'effet de la pression exercé sur le corps du flacon. En revanche, une solution à caractère moussant favorise la création de bulles de taille plus importante dont la particularité est de pouvoir se déformer avant de pouvoir éclater, ce qui leur donne un temps de vie plus long à l'intérieur de la tête de distribution.

En évitant les effets perturbateurs d'un mélange biphasique dans le liquide expulsé et les conséquences d'un dysfonctionnement de la membrane bifonctionnelle, l'invention permet d'assurer la reproductibilité du volume de liquide dans chaque goutte du liquide délivré à chaque opération de distribution ainsi que la composition de ce liquide. Il s'agit des deux facteurs essentiels pour que la dose d'administration prescrite soit respectée par un malade dans le cas de gouttes ophtalmiques. Ces avantages apportés par l'invention sont obtenus tout en préservant les conditions de bon fonctionnement qui sont liées aux conditions de circulation du liquide, ce d'autant mieux que l'on joue sur la circulation de l'air au niveau de son entrée dans le réservoir interne au flacon, donc en amont de la traversée du tampon poreux dans le sens de circulation du liquide.

D'une manière plus précise, le flacon suivant l'invention, destiné au conditionnement d'un liquide à distribuer goutte à goutte, présente avantageusement les particularités exposées ci-après, à considérer séparément ou dans toute combinaison techniquement réalisable, tant que le résultat entre dans le champ des revendications. Il comprend un réservoir à paroi à déformation élastiquement réversible par admission d'air à l'intérieur dudit réservoir par l'intermédiaire d'une tête de distribution par laquelle le liquide est délivré sous l'effet d'une pression exercée contre ladite paroi, ladite tête de distribution comportant un insert à corps évidé, par lequel elle se monte de manière étanche dans le col du flacon, en communication avec ledit réservoir. Cette tête de distribution comprend un embout la prolongeant à l'extérieur du flacon qui est percé d'un canal central débouchant par un orifice d'expulsion du liquide. Elle comprend aussi une membrane filtrante anti-bactérienne, réalisée partiellement hydrophile et partiellement hydrophobe, qui est montée à la base dudit embout, entre celui-ci et ledit insert. Elle autorise à travers elle le passage du liquide dans le sens de la distribution et le passage en sens inverse de l'air appelé à rentrer en compensation dans le flacon après une distribution de liquide. L'insert contient un tampon poreux régulateur de flux sur le trajet du liquide poussé du réservoir vers le canal d'expulsion (lors d'une diminution du volume interne sous l'effet de la pression exercée manuellement sur la paroi du flacon). Ce tampon, avantageusement réalisé en matériau hydrophobe, est placé en travers du conduit formé par l'évidemment central du tampon, dans une position axiale en aval du réservoir et en amont d'une chambre qui est elle-même délimitée en aval par la membrane filtrante disposée en amont de l'embout Les termes amont et aval se définissent ici par rapport au sens de circulation du liquide lors de sa distribution, c'est-à-dire du réservoir de conditionnement vers le canal d'expulsion des gouttes.

Selon l'invention, l'insert à corps évidé se termine avantageusement, à son extrémité dirigée vers le réservoir, par une embase formée par des arches longitudinales supportant une pastille centrale de diamètre inférieur au diamètre intérieur de l'insert, à distance de la périphérie interne du flacon au niveau de son espace interne constituant le réservoir récepteur du liquide au repos. Sa forme laisse un espace ouvert important pour le passage du liquide quittant le réservoir vers la tête de distribution, et surtout elle ménage des passages largement ouverts dans le flacon en direction radiale, ce qui facilite la rentrée de l'air compensatoire aspiré à travers le tampon poreux entre deux opérations d'expulsion de liquide, en le répartissant immédiatement sur toute la section du flacon, y compris au voisinage de sa paroi périphérique.

Les arches de la base de l'insert se présentent avantageusement sous forme de pattes longitudinales qui se replient radialement à leur extrémité inférieure pour supporter la pastille centrale et la relier à la couronne extérieure du corps. Une telle forme confère à l'insert une bonne résistance à la compression au niveau de sa base. Elle apporte une bonne résistance mécanique à l'insert dans le sens longitudinal évitant le flambage. Elle permet de guider cet ensemble, tout en lui apportant de la résistance mécanique, lors de sa mise en place par insertion dans le col du flacon par poussée longitudinale selon l'axe du col. Elle permet également de retenir le tampon poreux qui risquerait de glisser vers le réservoir du flacon lors des manipulations. En outre, elle contribue à assurer l'individualisation des éléments d'un ensemble de têtes de distribution pendant leur stockage et leur transport quand elles sont ensachées en vrac avant leur montage dans les flacons. L'encombrement des bases d'insert (arches et pastille centrale) empêche que deux têtes de distribution se trouvent liées ensemble par pénétration d'un embout de l'une dans le conduit central de l'insert de l'autre, et l'on évite ainsi la contrainte que représenterait, au niveau du procédé de fabrication industrielle des collures, le fait d'avoir à séparer des têtes devenues par là solidaires.

Le flacon muni d'une tête de distribution selon la présente invention s'avère très avantageux en terme de régulation de flux et de contrôle du volume des gouttes expulsées, en raison de la structure particulière de l'embase de l'insert placé dans le col du réservoir et contenant le tampon microporeux régulateur de flux. Une telle structure permet notamment un retour aisé de l'air dans le réservoir de stockage du liquide, en éloignement de la membrane, après chaque opération de distribution. Cette régularisation des flux d'air et de liquide diminue la quantité de bulles d'air dans le liquide qui s'écoule vers la membrane au moment de la distribution des gouttes de liquide, notamment des liquides tensioactifs. On dispose par ailleurs pour la chambre intermédiaire entre le tampon poreux et la membrane, d'une distance appropriée nécessaire à l'éclatement des bulles d'air qui peuvent être présentes à l'interface de sortie du tampon lorsque le flux de liquide entraîne avec lui de l'air qui est resté absorbé au sein du tampon entre deux opérations d'expulsion. On peut considérer que cette chambre a, pour le flux de liquide en sortie, un rôle de tampon régulateur de débit complémentaire à celui du tampon matériel, ainsi qu'un rôle répartiteur en sens transversal avant la traversée de la membrane si celle-ci présente une répartition homogène des zones hydrophiles et des zones hydrophobes. De plus ladite chambre faisant coussin d'air, elle assure aussi un rôle répartiteur en section transversale lors du fonctionnement en rentrée d'air entre l'embout qui admet l'air et la face externe du tampon matériel, ce qui favorise de bonnes conditions de rentrée d'air. Elle favorise la délivrance de doses régulières de liquide. Cette chambre assure aussi, comme cela est déjà connu antérieurement, le maintien de la membrane au sec entre deux opérations de distribution, lorsque le récipient repose dans sa position normale, c'est-à-dire en appui sur sa base, en recevant en son intérieur le liquide résiduel non expulsé.

Suivant des modes de réalisation préférés dans la pratique industrielle, l'invention répond en outre aux caractéristiques suivantes, mises en oeuvre séparément ou en chacune de leurs combinaisons techniquement opérantes.

Suivant une caractéristique avantageuse de l'invention, les arches sont au nombre de trois, disposées à équidistance les unes des autres, de manière à ouvrir largement la section de passage offerte à l'air pénétrant dans le flacon, tout en conférant une bonne solidité à la base du corps d'insert.

Dans le même objectif d'éviter toute perte de charge sur le flux d'air entrant dans le flacon, dans des modes de réalisation préférés de l'invention, les arches présentent une hauteur comprise entre 1 et 5 mm, en particulier entre 2 et 4 mm.

Le tampon microporeux hydrophobe situé dans le corps, dont il occupe toute la section, et à travers lequel il se crée une perte de charge, assure la régulation des flux de liquide. Il favorise en cela la distribution contrôlée des doses de liquide. Il empêche également le liquide contenu dans le réservoir de s'écouler lorsque aucune pression n'est exercée sur la paroi de celui-ci.

Suivant une caractéristique avantageuse de l'invention, le tampon est conçu pour emprisonner le minimum d'air dans ses pores.

La porosité de ce tampon est adaptée au flux entrant (air) et sortant (liquide) pour ne pas provoquer une perte de charge trop importante et doit également garantir une taille adéquate aux bulles d'air pour qu'elles puissent éclater facilement avant d'atteindre la surface de la membrane.

Ainsi, dans des modes de réalisation préférés de l'invention, le tampon microporeux hydrophobe présente une porosité équivalente comprise entre 20 et 120 µm.

Le tampon est de préférence constitué en polyéthylène basse densité, qui lui confère un caractère hydrophobe de sorte qu'il n'est pas mouillable. Son caractère microporeux autorise néanmoins le passage du liquide à travers lui, sous l'effet d'un différentiel de pression suffisant, induit entre le réservoir et l'extérieur par la pression exercée par l'utilisateur sur sa paroi déformable. Sa nature est telle qu'il ne présente pas d'interactions avec la formulation du liquide, plus particulièrement avec le principe actif.

Pour obtenir la hauteur de chambre (distance entre la face supérieure du tampon et la membrane) nécessaire à l'éclatement des bulles d'air avant qu'elles atteignent la membrane, on peut jouer sur l'épaisseur longitudinale (hauteur) et/ou la disposition du tampon.

Suivant une caractéristique avantageuse de l'invention, le tampon présente une épaisseur longitudinale qui est suffisamment importante pour constituer une perte de charge apte à empêcher le liquide de mouiller la membrane avant la première utilisation du flacon, afin d'éviter la détérioration de cette dernière, tout en étant suffisamment faible pour assurer que la chambre, ménagée entre lui et la membrane, présente une hauteur importante. Ainsi, lors de l'utilisation du flacon, les bulles pouvant se former sur la surface supérieure du tampon, c'est-à-dire la surface dirigée vers la chambre ont toute la place pour éclater à l'intérieur de la chambre sans risquer de venir atteindre la membrane bifonctionnelle. Cette dernière est ainsi bien préservée de ces bulles d'air.

Selon un mode préféré de l'invention, la hauteur de la chambre, soit la distance entre la face supérieure du tampon poreux et la membrane, est supérieure à 2 mm. De préférence elle est supérieure ou égale à environ 3 mm. De préférence encore, elle a une valeur comprise entre 4 et 10 mm, notamment entre 5 et 9 mm.

L'épaisseur longitudinale du tampon, une fois introduit dans l'insert, est de préférence comprise entre environ 0,3 et 0,8 mm. Le tampon est comprimé lors de sa mise en place dans le corps évidé et son épaisseur peut ainsi être diminuée de 50%. Il est disposé dans l'insert de manière à ce que la chambre qui la surmonte en aval (dans le sens de circulation du liquide) présente une hauteur suffisante, comme définie au-dessus, pour permettre aux bulles d'air qui peuvent se former dans le liquide en sortie du tampon d'éclater avant d'atteindre la membrane.

La membrane bifonctionnelle est classique en elle-même. Elle est par exemple réalisée en polymères à base de résine de polyamide ou de résine de polyéther sulfone. On lui donne un caractère hydrophile de base, de nature à autoriser sélectivement le passage du liquide à travers elle lors de l'opération de distribution. Elle est rendue partiellement hydrophobe sur une partie de sa surface, qui autorise alors sélectivement le passage de l'air de l'extérieur vers le réservoir après chaque opération de distribution, par modification de sa structure, notamment par greffage en présence d'un initiateur de réactions radicalaires. Ce traitement est notamment réalisé sur une bande médiane occupant 20 à 50 % de sa surface disposée au travers du chemin du liquide. La membrane présente en outre de préférence un diamètre de pores moyen de l'ordre de 0,1 à 0,2 µm, ceci afin de jouer un rôle anti-bactérien par filtration préservant le liquide encore présent dans le flacon de toute contamination biologique venant de l'extérieur.

Suivant une caractéristique avantageuse de l'invention, l'insert à corps évidé est conçu pour pouvoir être installé en force dans le col du flacon par poussée selon un axe longitudinal. Il est de préférence assemblé à l'intérieur du récipient par emboîtement en contact étroit.

La pastille centrale supportée par les arches à distance de la partie principale de l'insert, est avantageusement utile pour apporter de la résistance lors de l'introduction, réalisée en force, de l'insert dans le col du récipient lors de la fabrication de celui-ci. Elle est très utile dans le cas où le procédé de fabrication de l'insert se fait par injection de matière plastique, car elle permet de situer le point d'injection à son niveau et de prévenir des problèmes d'étanchéité que poseraient des bavures de moulage se situant sur le pourtour du corps d'insert.

Dans des modes de réalisation préférés de l'invention, le corps d'insert est étastiquement déformable à sa périphérie pour faciliter sa mise en place par insertion en force à l'intérieur dans le col du récipient. Il comporte sur son bord supérieur une couronne de plus large diamètre venant en appui contre le bord supérieur du col du récipient, de manière à assurer un bon positionnement de l'insert dans le col. Lors du montage, cette couronne sert également avantageusement d'appui pour la poussée de l'insert à l'intérieur du col.

L'insert est de préférence équipé sur sa surface extérieure d'au moins un joint torique, de préférence d'une pluralité de joints toriques axialement répartis, assurant l'étanchéité du contact entre l'insert et le col du récipient. Ces joints toriques, appelés godrons, constituent notamment un ensemble monobloc avec l'insert.

L'insert, comme les autres éléments constitutifs du récipient selon l'invention, sont de préférence avantageusement fabriqués par moulage, puis assemblés les uns aux autres.

Le récipient selon la présente invention offre en outre les avantages, tels qu'ils peuvent être décrits dans les brevets de la demanderesse et notamment du brevet publié sous le numéro FR 2 872 137, liés à la présence d'un réservoir à paroi à déformation réversible, d'une membrane filtrante anti-bactérienne, d'un tampon régulateur de flux et d'une chambre intercalaire entre ces deux derniers éléments. En particulier, la paroi à déformation réversible assure une utilisation optimale de tout le liquide contenu dans le réservoir: ce dernier conserve une capacité d'expulsion intacte tout au long des opérations de distribution successives, grâce à l'admission d'air qui ramène la pression d'expulsion à sa valeur initiale après chaque opération d'expulsion. La membrane protège le liquide contenu dans le réservoir des contaminations extérieures. Elle constitue également une cause de perte de charge, qui s'ajoute à celle générée par le tampon microporeux pour améliorer la régulation du flux de liquide, et pour assurer l'absence de fuites hors du réservoir quand aucune pression n'est exercée sur la paroi de ce dernier.

Le récipient selon l'invention, outre son efficacité en termes de contrôle du volume de liquide délivré, de conservation du liquide dans le réservoir et d'optimisation de l'utilisation de la totalité du volume de liquide, présente en outre les avantages d'une structure simple, d'un coût de fabrication limité et tout à fait approprié au domaine d'application des produits consommables, à jeter après usage, ainsi qu'une grande facilité d'utilisation pour le consommateur.

Comme indiqué, l'invention s'applique plus particulièrement à un flacon utilisé pour le conditionnement et la distribution d'un liquide contenant des composés présentant des propriétés tensio-actives, en particulier un collyre.

L'invention peut trouver application dans tous les domaines où l'on utilise des flacons compte-gouttes, et ce plus particulièrement dans toute situation où le produit à délivrer est un produit moussant, contenant un composé ayant des propriétés tensio-actives favorisant la formation de bulles d'air.

L'invention concerne aussi la tête de distribution telle que décrite précédemment, destinée à équiper des réservoirs à paroi élastique de flacons compte-gouttes.

L'invention sera maintenant plus complètement décrite dans le cadre de caractéristiques préférées et de leurs avantages, en faisant référence aux figures 1 à 3 dans lesquelles :
- la figure 1 représente en coupe axiale un flacon selon l'invention ;
- la figure 1A représente en éclaté le flacon en coupe axiale de la figure 1 ;
- la figure 2 illustre l'insert à corps évidé de la tête de distribution du flacon de la figure 1, en vue en perspective ;
- et la figure 3 montre l'insert de la figure 2 en vue de dessus.

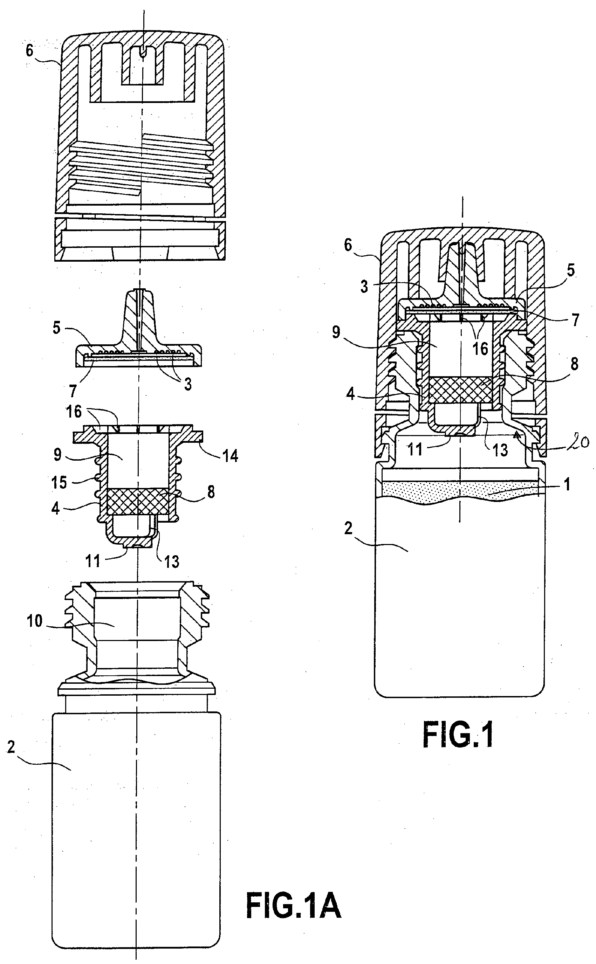

Un récipient de conditionnement d'un liquide à délivrer goutte à goutte est illustré sur les figures 1 et 1A sous la forme d'un flacon destiné plus particulièrement au conditionnement d'un collyre. Ses éléments sont globalement constitués en une matière plastique compatible avec l'application pour la conservation d'une solution ophtalmique. Ils sont notamment réalisés chacun en polymère de la famille des polyéthylènes.

Le flacon comporte un réservoir de stockage 2 du liquide dont la paroi périphérique cylindrique est à déformation élastiquement réversible, pour permettre une distribution du liquide 1 à partir d'une compression manuelle exercée sur elle par l'utilisateur, puis un retour spontané à sa forme initiale par admission d'air lorsque cette compression est relâchée. La rentrée d'air en compensation de chaque goutte de liquide expulsée s'effectue selon le trajet inverse de cette expulsion à travers la tête de distribution occupant le col du flacon. Aucune autre rentrée d'air n'est possible ; en particulier, il n'existe pas de trou d'équilibrage de pression à travers la paroi du flacon à la base du col. La paroi périphérique cylindrique présente à son extrémité libre une portion d'étranglement 20 qui se prolonge par un col 10.

La tête de distribution du liquide en goutte à goutte comprend un insert à corps évidé 4, disposé à l'intérieur du col 10 du flacon, un embout (ou buse) de distribution 5, et le cas échéant comme ici un capuchon amovible 6 d'obturation de l'embout, qui se visse de façon classique autour du col du flacon. Elle comprend aussi un tampon microporeux 8 inclus dans lé corps évidé 4 et une membrane anti-bactérienne 7 disposée à la base de l'embout 5.

La membrane filtrante anti-bactérienne 7, partiellement hydrophile et partiellement hydrophobe, est disposée en amont de l'embout 5, dans le sens de la circulation du liquide depuis le réservoir vers l'embout de distribution, pour protéger par filtration le liquide 1 des contaminations extérieures, notamment des bactéries. Cette membrane 7 est librement supportée en fonctionnement par application contre l'embase de l'embout 5. Elle est fixée sur son pourtour par soudage thermique entre une couronne périphérique de cette embase (qui présente là un bourrelet qui s'estompe lors de l'opération de soudage entre les deux pièces) et une portée coopérante en face terminale de l'insert 4. Elle est par exemple constituée en polyéther sulfone, rendu partiellement hydrophobe sur une partie de sa surface. Elle présente une porosité de l'ordre de 0,1 à 0,2 µm. L'embase de l'embout a la forme d'un disque évidé qui s'emboîte sur le corps évidé 4 ; elle comporte sur sa face interne des microcanaux 3 qui facilitent le drainage du liquide vers l'orifice d'expulsion.

L'insert 4 a une forme globalement cylindrique, ici d'un diamètre interne d'environ 1 cm, qui s'évase dans sa partie terminale supérieure pour atteindre un diamètre d'environ 1,5 cm pour former la portée coopérante, qui est munie d'ailettes 16 pour supporter la membrane 7 lorsqu'elle est plaquée entre l'insert et l'embout. Il a dans cet exemple une hauteur d'environ 1,5 cm en son corps principal (sans la base formée par les arches et la pastille). Il loge dans son évidement intérieur un tampon microporeux 8, de forme cylindrique épousant celle de cet évidement, qui est réalisé en une matière hydrophobe. Il est notamment constitué en un feutre à trame en polyéthylène. Par sa présence il a pour effet de réguler le flux de liquide distribué et d'interdire le passage du liquide du réservoir 2 vers l'embout 5 en l'absence d'une compression de la paroi du récipient, et pour réguler le flux.de liquide distribué. Ce tampon 8 est disposé en amont et à distance de la membrane 7, dans le sens de la circulation du liquide depuis le réservoir vers l'embout de distribution, de manière à ménager entre lui et cette dernière une chambre intercalaire 9. La chambre 9 permet notamment aux bulles d'air du liquide distribué d'éclater avant d'atteindre la membrane 7. Elle permet aussi de recueillir le liquide résiduel non expulsé.

Le tampon 8 présente une porosité équivalente d'environ 100 µm. Il présente également une hauteur, ici d'environ 0,5 cm à l'état comprimé quand il est en place dans l'insert 4, soit égale à environ 33% de la hauteur du corps d'insert. Avant d'y être introduit, le tampon a une hauteur d'environ 1 cm. Il est disposé dans la partie inférieure du corps d'insert, de manière à ce que la chambre 9 ménagée entre lui et la membrane 7 présente une hauteur importante, ici d'environ 6 mm. Les bulles d'air pouvant être emprisonnées dans le tampon après aspiration d'air consécutive à une opération d'expulsion d'une goutte de liquide, ont une taille telle que lorsqu'elles sont éjectées du tampon lors d'une opération de distribution du liquide ultérieure, en même temps que le liquide, elles éclatent dans la chambre intercalaire 9, avant d'arriver à la membrane bifonctionnelle.

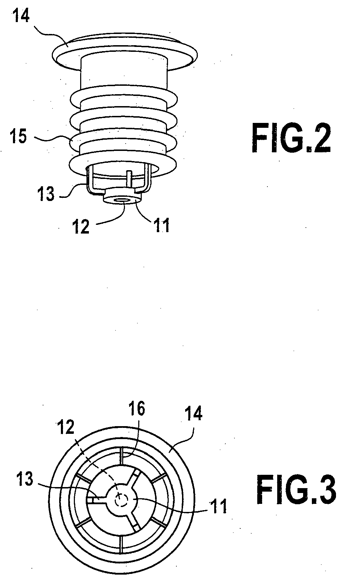

L'insert 4 comporte à sa base, à l'extrémité dirigée vers l'intérieur du réservoir contenant le liquide, comme illustré de façon plus détaillée sur la figure 2, une pastille centrale 11 de diamètre largement inférieur au diamètre de la partie cylindrique principale de l'insert, ici d'environ 0,4 cm. Cette pastille est supportée par des arches longitudinales 13. Ces arches sont sous forme de pattes relativement longues et spécifiquement orientées dans la direction longitudinale, qui rejoignent à une extrémité la partie cylindrique de l'insert 4, et qui en leur extrémité opposée se recourbent radialement vers l'axe de l'insert de manière à soutenir la pastille 11 centrée sur cet axe.

Les arches 13 présentent une hauteur suffisante pour ménager entre elles des passages largement ouverts à la circulation de l'air pénétrant dans le réservoir du flacon par le conduit axial de la tête de distribution. Cette hauteur est notamment environ égale à 3,5 mm. Les pattes qui les constituent ont chacune une longueur totale d'environ 5 mm. Les arches offrent un espace à la base de l'insert d'un volume d'environ 0,40 cm³, d'où l'air s'échappe principalement dans le sens radial tout autour de l'axe de l'ensemble. On comprend qu'il importe que les passages formés entre les arches permettent d'une part la circulation de liquide depuis le réservoir vers l'extérieur via le tampon de manière à ce que la base n'entrave pas la distribution de liquide, et que ces passages permettent d'autre part d'orienter le retour d'air vers les parois du réservoir. Ainsi, le retour d'air est orienté radialement sur les parois et non directement sur le liquide encore présent dans le réservoir. La pastille centrale présente à cet effet une forme de disque dont le diamètre est suffisamment grand pour bloquer le passage d'air axial en provenance du tampon, et ainsi diriger l'air entrant dans le réservoir vers les parois de celui-ci. Comme cela est visible sur la figure 1, il est particulièrement avantageux que lorsque l'insert est monté dans le col du flacon, la pastille centrale soit disposée au niveau de la portion d'étranglement 20 du flacon dans laquelle la paroi périphérique n'est pas essentiellement verticale. L'air entrant qui s'écarte radialement après obstruction de la pastille arrive sur cette partie inclinée de la paroi périphérique du flacon, de sorte que l'air entrant n'impacte pas frontalement la paroi mais est guidé dans un flux laminaire le long de cette paroi vers le liquide encore présent dans le réservoir.

Comme on peut le voir sur la figure 3, les arches 13 sont an nombre de trois, et disposées à équidistance les unes des autres de manière à assurer la solidité de cette partie de l'insert, notamment en terme de résistance à la compression dans la direction longitudinale. ll sera compris que le nombre des arches et leur disposition peuvent être différents dès lors qu'elles ménagent des passages ouverts à la circulation de liquide pour son expulsion et à la circulation de retour d'air,

La pastille centrale 11 facilite l'introduction en force de l'insert dans le col 10 du flacon. Elle comporte en son centre sur sa face externe un point 12 résultant du point d'injection de la matière polymère pour former l'ensemble. La pastille centrale a un rôle de butée lors de l'insertion de l'insert dans le col du flacon, tout en permettant avec les arches de faciliter le guidage axial de cet insert. Elle évite d'abîmer et d'enlever le tampon poreux lors du transport en vrac des têtes de distribution munies de leur embout. Sa présence est également utile dans le cadre du procédé préféré de fabrication de l'insert, par moulage par injection. Elle comporte en 12 la trace du point d'injection du polymère dans le moule. On évite qu'un point d'injection qui se trouverait sur un côté externe de l'insert puisse créer un point de fuite néfaste à l'étanchéité nécessaire entre l'insert et le col du flacon.

Sur le bord supérieur de l'insert 4 est formée une couronne périphérique 14 de plus grand diamètre, ici d'environ 2 cm. Cette couronne joue le rôle d'une butée permettant un positionnement adéquat de l'insert 4 dans le col 10 du flacon lors de l'assemblage.

Lors du montage, l'insert 4 est assemblé par emboîtement en force à l'intérieur du col 10 du flacon. Ceci est rendu possible par la légère capacité de déformation élastique du matériau constituant l'insert. Cet emboîtement est réalisé par l'intermédiaire de joints toriques circulaire 15, appelés godrons, ménagés à la périphérie de l'insert. Ces joints sont de préférence réalisés en monobloc avec l'insert, dans la même étape de fabrication par moulage. Ils assurent l'étanchéité de contact avec la paroi interne du col 10. Ils assurent aussi le guidage de l'insert au moment du montage par pression axiale, de manière à conduire globalement à un emboîtement en contact étroit sans risque de positionnement en biais.

On réalise des tests du flacon selon l'invention décrit avec une solution tamponnée comprenant un principe actif solubilisé dans un agent solubilisant ayant des propriétés tensio-actives pour vérifier la régularité du calibrage de la goutte de solution expulsée en fonction de la: hauteur de la chambre, pour une configuration d'insert et un tampon poreux donnés, répondant aux spécifications indiquées précédemment. La régularité du calibrage de la goutte est vérifiée en pesant dix gouttes expulsées et en répétant trois fois chaque essai. Ces essais montrent que pour une chambre ayant une hauteur d'au moins 3 mm, la variation du volume de la goutte expulsée est inférieure à 10 %, ce qui reste d'une qualité suffisante en pratique dans la plupart des situations. Pour une hauteur de 6 mm, les résultats sont encore meilleurs, avec une variation inférieure à 5 %.

La description qui précède explique clairement comment l'invention permet d'atteindre les objectifs qu'elle s'est fixés. En particulier, elle fournit un récipient de conditionnement et de distribution d'un liquide, notamment d'un liquide ophtalmique, du type à rentrée d'air et à membrane filtrante bifonctionnelle, qui assure une bonne régularité de la distribution, en évitant notamment la présence d'air dans les gouttes distribuées même dans les cas où le liquide présente des propriétés tensioactives. L'invention permet de maîtriser le bon calibrage de la goutte ainsi que la répétabilité de la dose distribuée afin de garantir la bonne efficacité du médicament et l'absence des effets secondaires que pourrait provoquer un dosage inapproprié. Mais bien entendu l'invention n'est pas limitée aux modes de mise en oeuvre qui ont été spécifiquement décrits et elle s'étend au champ défini dans les revendications.

## Revendications

1. Flacon de conditionnement d'un liquide à distribuer goutte à goutte comprenant un réservoir à paroi à déformation élastiquement réversible par admission d'air à l'intérieur dudit réservoir par l'intermédiaire d'une tête de distribution par laquelle le liquide est délivré sous l'effet d'une pression exercée contre ladite paroi,
dans lequel ladite tête de distribution comporte un insert à corps évidé, par lequel elle se monte de manière étanche dans un col du flacon, en communication avec ledit réservoir, et un embout de distribution dépassant en prolongement dudit corps à l'extérieur du flacon, qui est percé d'un canal central débouchant par un orifice d'expulsion du liquide,
ainsi qu'une membrane filtrante anti-bactérienne, partiellement hydrophile et partiellement hydrophobe, qui est montée en travers dudit insert à la base dudit embout, pour autoriser le passage du liquide dans le sens de la distribution et en sens inverse le passage de l'air appelé à rentrer en compensation dans le flacon après une distribution de liquide,
ledit insert contenant un tampon poreux régulateur du flux de liquide le traversant en direction de la membrane,
**caractérisé en ce que** l'insert (4) se termine à l'intérieur dudit réservoir, au-delà dudit tampon, par une base formant des arches longitudinales (13) supportant une pastille centrale (11) qui ménagent entre elles des passages ouverts à une circulation radiale, vers la périphérie du réservoir, pour l'air pénétrant dans le flacon après avoir traversé ledit tampon.

2. Flacon selon la revendication 1, **caractérisé en ce que** ladite pastille centrale est de diamètre inférieur au diamètre
intérieur de l'insert (4) et centrée dans l'axe du flacon, de manière à laisser un espace ouvert pour le passage de l'air autour d'elle tout en faisant obstacle à un jet axial direct en sortie de l'insert.

3. Flacon selon la revendication 2, **caractérisé en ce que** l'insert (4) est monté dans le col (10) du flacon avec sa base qui s'étend au niveau d'une portion d'étranglement (20) de la paroi périphérique du flacon, de sorte que l'air entrant, qui est dirigé radialement vers la paroi périphérique du flacon par obstruction de la pastille centrale, est guidé dans un flux laminaire le long de ladite paroi.

4. Flacon selon l'une des revendications 1 à 3, **caractérisé en ce que** lesdites arches (13) sont au nombre de trois, disposées à équidistance angulaire les unes des autres autour de l'axe du col du flacon.

5. Flacon selon l'une quelconque des revendications 1 à 4, **caractérisé en ce que** le tampon poreux est un tampon microporeux en matière hydrophobe sous la forme d'un feutre présentant un diamètre de pores équivalent compris entre 20 et 120 µm.

6. Flacon selon l'une quelconque des revendications 1 à 5, **caractérisé en ce que** la distance entre la face supérieure du tampon poreux (8) et la membrane (7) est supérieure à 2 mm, de préférence supérieure ou égale à environ 3 mm.

7. Flacon selon la revendication 6, **caractérisé en ce que** la distance entre la face supérieure du tampon poreux (8) et la membrane (7) est comprise entre 4 et 10 mm, de préférence entre 5 et 9 mm.

8. Flacon selon l'une quelconque des revendications 1 à 7, **caractérisé en ce que** les arches de l'insert (13) présentent une hauteur comprise entre 1 et 5 mm, de préférence entre 2 et 4 mm.

9. Flacon selon l'une quelconque des revendications 1 à 8, **caractérisé en ce que** l'insert (4) est assemblé dans le flacon par emboîtement en force à l'intérieur du col (10) du flacon et **en ce qu'**il comporte sur son bord supérieur une couronne (14) de plus large diamètre venant en appui contre le bord supérieur du col (10) du flacon de manière à assurer un bon positionnement dudit insert (4) dans ledit col (10).

10. Flacon selon la revendication 8 ou 9, **caractérisé en ce que** la surface extérieure de l'insert (4) est équipée d'une pluralité de joncs toriques axialement répartis.

11. Flacon selon l'une quelconque des revendications 1 à 7, **caractérisé en ce que** le tampon poreux (8) est réalisé en polyéthylène basse densité.

12. Flacon selon l'une quelconque des revendications précédentes, **caractérisé en ce qu'**il est utilisé pour le conditionnement d'un liquide contenant des composés présentant des propriétés tensio-actives, en particulier un collyre.

## Patentansprüche

1. Tropffläschchen zum Aufbewahren einer Flüssigkeit, wobei das Tropffläschchen ein Reservoir mit reversibel verformbarer elastischer Wand durch Einlassen von Luft in das Innere des Reservoirs über einen Verteilerkopf umfasst, durch den die Flüssigkeit unter der Wirkung eines gegen die Wand ausgeübten Drucks abgegeben wird,
wobei der Verteilerkopf einen Hohlkörpereinsatz aufweist, durch den er an einem Hals des Fläschchens kommunizierend mit dem Reservoir befestigt ist und wobei ein Verteilerflansch in Verlängerung des Körpers zum Äußeren des Fläschchens hinausragt, der von einem zentralen Durchgang durchbohrt ist, der in eine Ausstoßöffnung der Flüssigkeit mündet,
sowie eine antibakterielle, teilweise hydrophile und teilweise hydrophobe Filtrationsmembran, die quer zu dem Einsatz am Fuße des Flansches befestigt ist, um den Durchgang der Flüssigkeit in Verteilungsrichtung und in umgekehrter Richtung den Durchgang von eingesaugter Luft zum Ausgleich in das Fläschchen nach einer Flüssigkeitsverteilung zu ermöglichen,
wobei der Einsatz einen porösen Regulierungspfropfen des ihn in Richtung der Membran durchlaufenden Flüssigkeitsflusses aufweist,
**dadurch gekennzeichnet, dass** der Einsatz (4) im Inneren des Reservoirs oberhalb des Pfropfens in einer Basis endet, die Längsbögen (13) ausbildet, die ein zentrales Plättchen (11) tragen, die zwischen ihnen zu einer radialen Zirkulation hin in Richtung des Umfangs des Reservoirs geöffnete Durchgänge für die Luft, die nach dem Durchlauf durch den Pfropfen in das Fläschchen dringt, schaffen.

2. Fläschchen nach Anspruch 1, **dadurch gekennzeichnet, dass** das zentrale Plättchen einen Durchmesser aufweist, der kleiner ist als der Innendurchmesser des Einsatzes (4), und das um die Achse des Fläschchens so zentriert ist, dass ein Raum offen gelassen wird für den Durchgang der Luft um sie herum unter Schaffung eines Hindernisses für einen direkten axialen Strahl am Ausgang des Einsatzes.

3. Fläschchen nach Anspruch 2, **dadurch gekennzeichnet, dass** der Einsatz (4) im Hals (10) des Fläschchens angeordnet ist, wobei sich seine Basis an einem Drosselteil (20) der Umfangswand des Fläschchens erstreckt, derart dass eintretenden Luft, die radial zur Umfangswand des Fläschchens durch Versperren des zentralen Plättchens geleitet wird, in einem laminaren Fluss entlang der Wand geführt wird.

4. Fläschchen nach einem der Ansprüche 1 bis 3, **dadurch gekennzeichnet, dass** drei Bögen (13) vorhanden sind, die voneinander in Winkeläquidistanz um die Achse des Halses des Fläschchens angeordnet sind.

5. Fläschchen nach einem der Ansprüche 1 bis 4, **dadurch gekennzeichnet, dass** der poröse Pfropfen ein mikroporöser Pfropfen aus hydrophobem Material in der Form eines Filzes ist, der einen entsprechenden Poren-Durchmesser von 20 bis 120 µm aufweist.

6. Fläschchen nach einem der Ansprüche 1 bis 5, **dadurch gekennzeichnet, dass** der Abstand zwischen der oberen Fläche des porösen Pfropfens (8) und der Membran (7) größer als 2 mm, vorzugsweise größer als oder gleich etwa 3 mm ist.

7. Fläschchen nach Anspruch 6, **dadurch gekennzeichnet, dass** der Abstand zwischen der oberen Fläche des porösen Pfropfens (8) und der Membran (7) 4 bis 10 mm, vorzugsweise von 5 bis 9 mm beträgt.

8. Fläschchen nach einem der Ansprüche 1 bis 7, **dadurch gekennzeichnet**, das die Bögen des Einsatzes (13) eine Höhe von 1 bis 5 mm, vorzugsweise zwischen 2 und 4 mm aufweisen.

9. Fläschchen nach einem der Ansprüche 1 bis 8, **dadurch gekennzeichnet, dass** der Einsatz (4) in das Fläschchen durch Presssitz in das Innere des Halses (10) des Fläschchens eingefügt ist und dadurch, dass er an seiner Oberkante einen Kranz (14) von größerem Durchmesser aufweist, der auf dem oberen Rand des Halses (10) des Fläschchens so aufliegt, dass eine gute Positionierung des Einsatzes (4) in dem Hals (10) sichergestellt ist.

10. Fläschchen nach Anspruch 8 oder 9, **dadurch gekennzeichnet, dass** die äußere Fläche des Einsatzes (4) mit einer Vielzahl von axial verteilten torischen Ringen versehen ist.

11. Fläschchen nach einem der Ansprüche 1 bis 7, **dadurch gekennzeichnet, dass** der poröse Pfropfen (8) aus Polyethylen mit geringer Dichte hergestellt ist.

12. Fläschchen nach einem der vorangehenden Ansprüche, **dadurch gekennzeichnet, dass** es zur Aufbewahrung einer Flüssigkeit verwendet wird, die Verbindungen enthält, die grenzflächenaktive Eigenschaften aufweisen, insbesondere Augentropfen.

## Claims

1. Vial for the packaging of a liquid to be dispensed drop by drop, comprising a reservoir having a wall which can be reversibly resiliently deformed by admission of air into said reservoir by way of a dispensing head by means of which the liquid is delivered under the effect of a pressure exerted against said wall,
wherein said dispensing head comprises an insert with a hollow body, by means of which it is mounted in a sealed manner in a neck of the vial, in communication with said reservoir, and a dispensing tip which projects as a continuation of said body outside the vial and which is provided with a central channel which opens in a liquid expulsion orifice,
as well as an antibacterial filtration membrane, which is partially hydrophilic and partially hydrophobic and which is mounted across said insert at the base of said tip in order to permit the passage of the liquid in the dispensing direction and, in the opposite direction, the passage of the air which is made to return by way of compensation into the vial after liquid has been dispensed,
said insert containing a porous pad which regulates the flow of liquid passing through it in the direction of the membrane,
**characterised in that** the insert (4) terminates inside said reservoir, beyond said pad, in a base forming longitudinal arches (13) which support a central disc (11) and form between them passages which are open for radial circulation, towards the periphery of the reservoir, of the air that enters the vial after passing through said pad.

2. Vial according to claim 1, **characterised in that** said central disc has a diameter smaller than the inside diameter of the insert (4) and is centred on the axis of the vial, so as to leave an open space for the passage of the air around it, while forming an obstacle to a direct axial jet coming from the insert.

3. Vial according to claim 2, **characterised in that** the insert (4) is mounted in the neck (10) of the vial with its base which extends at the level of a narrowed portion (20) of the peripheral wall of the vial, so that the incoming air, which is directed radially towards the peripheral wall of the vial by the obstruction formed by the central disc, is guided in a laminar flow along said wall.

4. Vial according to any one of claims 1 to 3, **characterised in that** there are three arches (13), which are arranged at equal angular distances from one another about the axis of the neck of the vial.

5. Vial according to any one of claims 1 to 4, **characterised in that** the porous pad is a microporous pad of hydrophobic material in the form of a felt having an equivalent pore diameter of from 20 to 120 µm.

6. Vial according to any one of claims 1 to 5, **characterised in that** the distance between the upper face of the porous pad (8) and the membrane (7) is greater than 2 mm, preferably greater than or equal to approximately 3 mm.

7. Vial according to claim 6, **characterised in that** the distance between the upper face of the porous pad (8) and the membrane (7) is from 4 to 10 mm, preferably from 5 to 9 mm.

8. Vial according to any one of claims 1 to 7, **characterised in that** the arches of the insert (13) have a height of from 1 to 5 mm, preferably from 2 to 4 mm.

9. Vial according to any one of claims 1 to 8, **characterised in that** the insert (4) is mounted in the vial by force-fitting inside the neck (10) of the vial, and **in that** it comprises on its upper edge a ring (14) of larger diameter which rests against the upper edge of the neck (10) of the vial so as to ensure correct positioning of said insert (4) in said neck (10).

10. Vial according to claim 8 or 9, **characterised in that** the outer surface of the insert (4) is provided with a plurality of axially distributed O-rings.

11. Vial according to any one of claims 1 to 7, **characterised in that** the porous pad (8) is made of low-density polyethylene.

12. Vial according to any one of the preceding claims, **characterised in that** it is used for the packaging of a liquid containing compounds having surface-active properties, in particular eye drops.
